# EUROPEAN PATENT SPECIFICATION

(11) **EP 2 825 229 B1**
(45) Date of publication and mention of the grant of the patent: **30.12.2020**
(21) Application number: 13708774.8
(22) Date of filing: 07.03.2013
(51) Int. Cl.: A61M 15/00, A61M 11/00, A61M 5/24, A61M 5/315

(54) **MEDICAMENT DELIVERY DEVICE**
VORRICHTUNG ZUR MEDIKAMENTENVERABREICHUNG
DISPOSITIF D'ADMINISTRATION DE MÉDICAMENT

(30) Priority: 16.03.2012 SE 1250249; 16.03.2012 US 201261612012 P
(43) Date of publication of application: 21.01.2015
(73) Proprietor: SHL Medical AG, 6302 Zug (CH)
(72) Inventor: NOREBRING, Jonas, 11823 Stockholm (SE)
(86) International application number: PCT/EP2013/054577
(87) International publication number: WO 2013/135549

(56) References cited:
- EP-A2- 0 793 973
- EP-A2- 0 911 046
- WO-A1-2007/094833
- WO-A1-2011/139212
- US-A- 3 110 309
- US-A- 4 613 326
- US-B1- 6 440 105

## Description

### Field of the invention

The present invention relates to an medicament delivery device intended for use in combination with with a disposable container

### Background of the invention

Disposable containers as multi-chamber containers or dual-chamber containers are frequently used in medicament delivery devices in order to facilitate the distribution of drugs to a patient. Dual-chamber containers comprise two longitudinally arranged chambers divided by a movable plunger arranged in the container. In one of chambers, normally the proximal chamber, a predetermined amount of freeze-dried drug is arranged while a diluent is arranged in the distal chamber. When a distal plunger is exposed to a pushing force, the diluent is transferred via a bypass passage to the proximal chamber where the diluent is mixed with the freeze-dried drug. When the distal plunger reaches the dividing proximal plunger, the distal chamber is completely empty and the mixture in the proximal chamber is ready for delivery. Continued proximal movement of the two plungers will deliver the mixture in the proximal chamber via a delivery member i.e. a needle, a nozzle, a mouth piece , etc. to the patient.

Unfortunately, there is a risk that a small amount of the mixture is encapsuled in the bypass passage and may flow back, distally via the bypass passage before the opening of the bypass passage is completely closed by the distal plunger. If the medicament delivery device is held in a vertical position with the delivery member pointing upwards, which is the normal position during preparation of medicament delivery devices i.e. injectors, inhalers, etc. the leaking mixture could continue flowing distally downwards along the plunger rod to the driving mechanism and affect the mechanism adversely. Leakage may sometimes also occur during use of conventional single-chamber containers but not as often as when dual-chamber containers are used. Medicament delivery devices with different kind of leakage prevention means are for example disclosed in US 6 440 105, EP 0 911 046, EP 0 793 973, US 4 613 326 and WO 2007/094833.

There is consequently a need for an improved medicament delivery device that can be used over a long period of time together with disposable containers without risking adverse effects from fluids leaking from the containers.

### Summary of the invention

The present invention, defined in the appended claims, provides a medicament delivery device that fulfils the needs defined above.

The medicament delivery device according to the invention is elongated along a longitudinal axis (A), has a proximal end and a distal end. Said device is intended for use in combination with a disposable container which comprises at least one chamber for a liquid medicament encapsulated in the container by at least one movable plunger, said device comprises:
- an elongated plunger rod configured to be in contact with the at least one movable plunger;
- a drive mechanism configured to, when the medicament delivery device is activated, apply a force in a proximal direction via said plunger rod acting on said at least one movable plunger;
- an elogated housing comprising a proximal housing section and a distal housing section, said proximal housing section being configured to accomodate the container and said distal housing section being configured to accomodate the plunger rod and the driving mechanism. Said elongated plunger rod comprises an outer periphery in which at least two axially separated recesses and/or cut-out portions are arranged for retaining leaking liquid medicament that may flow along the plunger rod to the drive mechanism.

The claimed invention fulfils the needs defined above since fluid leaking from the container is trapped in the recesses and/or cut-out portions in the plunger rod and is thereby prevented from reaching the drive mechanism in the distal end of the medicament delivery device.

In one embodiment of the invention, the disposable container is a multi-chamber medicament container comprising a proximal chamber wherein an active medicament is encapsulated, a distal chamber wherein a diluent is encapsulated, a passage between the chambers, a proximal movable plunger sealing the proximal chamber and the passage, and a distal movable plunger sealing the distal chamber. The proximal end of elongated plunger rod is configured to be in contact with the distal movable plunger.

In one embodiment of the invention, the device is configured such that when a force is exerted on said distal movable plunger by the plunger rod which is driven by the drive mechanism, said distal and proximal plungers are forced to move towards the proximal end of the device such that the diluent may flow through the passage for being mixed with the active medicament forming a liquid medicament.

In one embodiment of the invention, a spinner is arranged rotatable around the longitudinal axis and in relation to the plunger rod, at the proximal end of said plunger rod, said spinner having an outer periphery also provided with one, or more, recesses and/or cut-out portions. This embodiment is favourable since recesses and/or cut-out portions of the spinner are located close to the movable plunger and the inner periphery of the container and will provide a first efficient trap for leaking fluid.

In one embodiment of the invention, the axially separated recesses and/or cut-out portions of the plunger rod are arranged at different locations around the outer periphery of the plunger rod. This embodiment provides an even more efficient trap for the liquid since the different locations of the recesses and/or cut-out portions will reduce the risk that leaking fluid is able to continue along the plunger rod between adjacent recesses and/or cut-out portions.

In one embodiment of the invention, said at least two recesses and/or cut-out portions in said plunger rod extend a radial distance from the outer periphery of the plunger rod towards the longitudnal axis (A) that is less then the radius of the plunger rod in the area of the recess or cut-out portion. This embodiment is favourable since the center of the plunger will remain solid through the entire plunger rod. This ensures that the plunger rod will have the required axial strength to withstand the force applied by the drive mechanism on the plunger rod.

In one embodiment of the invention, said recesses and/or cut-out portions in the plunger rod and/or spinner are filled with a liquid-absorbing material. This embodiment further improves the posibillity for the plunger rod to absorb and maintain the leaking fluid in the recesses and/or cut-out portions.

In one embodiment of the invention, the peripheral width of said recesses and/or cut-out portions in the plunger rod exceeds half the peripheral width of the plunger rod in the area of the recesses and/or cut-out portions. This embodiment is favourable since larger recesses and/or cut-out portions will increase the possibilities of preventing fluid from continuing along the plunger rod.

The medicament delivery device is an injector or an inhaler.

The different embodiments described above could of course be combined and modified in different ways without departing from the scope of the invention that is defined by the claims. Further aspects of the invention will be described more in detail in the detailed description.

### Brief description of the drawings

One embodiment of the present invention is illustrated in the appended figures disclosing:
Figure 1 is an exploded perspective side view.

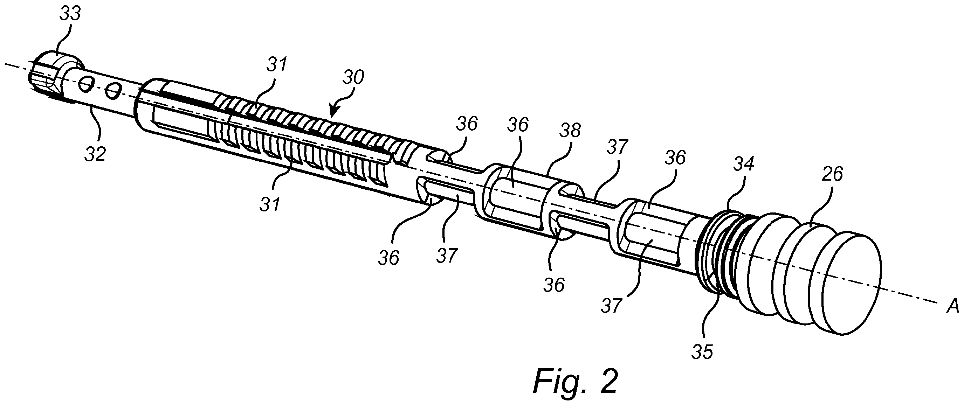

Figure 2 illustrates the plunger rod according to the invention.
Figure 3a illustrates the proximal housing section before fluid in the second chamber of the container is mixed with the drug in the first chamber of the container.
Figure 3b illustrates the proximal housing section and the container after mixing, ready for use.
Figure 3c illustrates the position of the plunger rod when the medicament delivery has been started but not ended.
Figure 3d illustrates the plunger rod and the container once the medicament delivery is completed.

### Detailed description

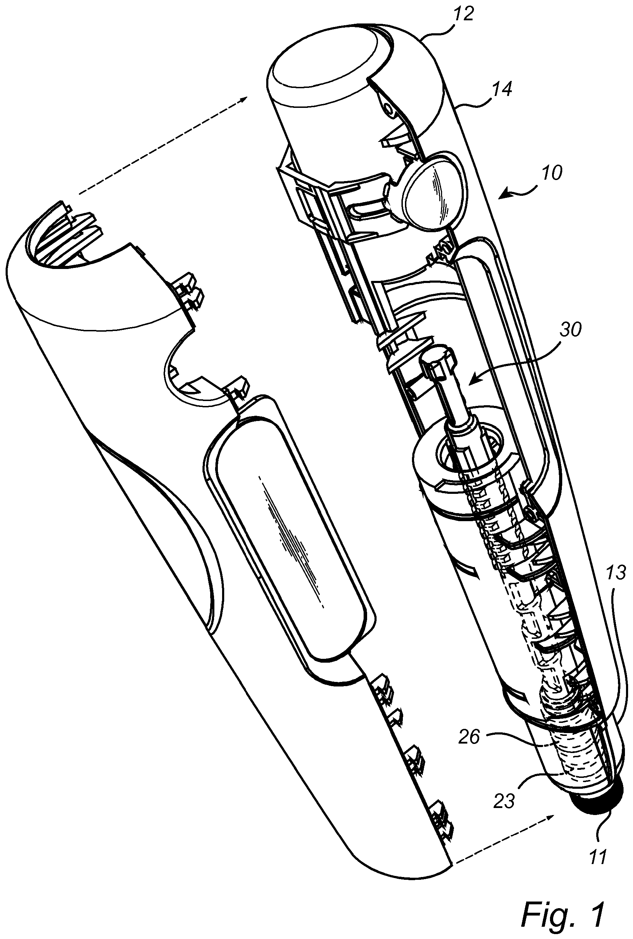

In figure 1, one embodiment of an medicament delivery device according to the present invention is schematically illustrated in an exploded perspective view in order to illustrate the different components of the medicament delivery device 10. The medicament delivery device 10 is mostly intended for self administration of a drug by a patient. The medicament delivery device 10 comprises a protective elongated housing having a proximal 11 and distal end 12, and a longitudinal axis (A). The medicament delivery device housing is divided in a proximal housing section 13 and a distal housing section 14 that during the assembly of the medicament delivery device 10 are removably attached to each other. The medicament delivery device 10 furthermore comprises a movable plunger rod 30 driven by a drive mechanism, not illustrated, arranged in the distal housing section 14. The medicament delivery device 10 is intended for use in combination with disposable containers 20, preferably disposable multi-chamber containers, that during the assembly of the medicament delivery device 10 are arranged in the proximal housing section 13.

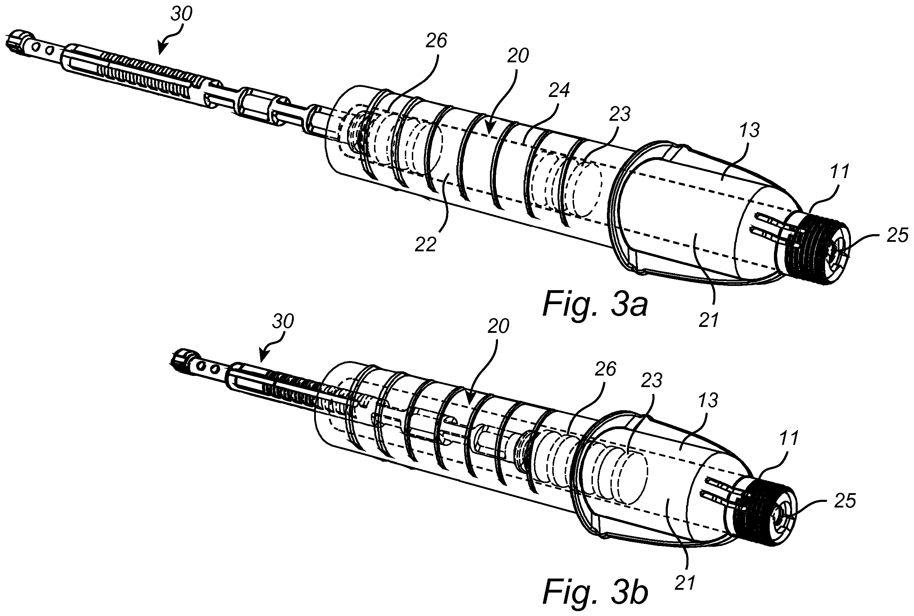

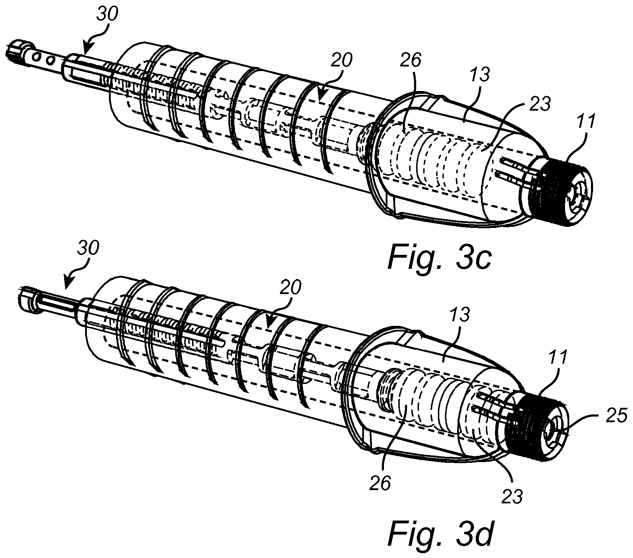

Within the proximal housing section 13, a space is defined in order to house the disposable container 20. The disposable container 20 is either a dual chamber container, as illustrated in the figures 3a -3d, or a conventional one-chamber container. The container 20 comprises a container vessel 24 normally made of glass and sealed in the proximal end by an elastic sealing member 25. The elastic sealing member 25 is punctuated by a delivery member i.e. a needle, not illustrated in the figures, when the medicament delivery device 10 is prepared for use. The container is sealed by a distal movable plunger 26 at the distal end of the container vessel in order to seal the container. The illustrated container is a dual-chamber container, illustrated for example in figure 3a to 3d. Dual-chamber containers comprise two longitudinally arranged chambers, a proximal chamber 21 and a distal chamber 22, divided by a proximal movable plunger 23 arranged in the container. When the medicament delivery device 10 is activated and prepared for use, the distal plunger 26 is moved in the proximal direction by the movable plunger rod 30 which is driven by the drive mechanism such that a diluent from the distal chamber 22 is transferred via a bypas passage to the proximal chamber 21 whereby the diluent is mixed with a freeze-dried drug, figure 4b, forming a liquid medicament. When the distal plunger 26 reaches the proximal plunger 23, the distal chamber 22 is emptied completely and the mixture in the proximal chamber 21 is ready for use.

Continued distal movement of the plungers 26 and 23 will deliver the liquid medicament in the proximal chamber 21, figure 3c, to the patient via a non-illustrated delivery member arranged at the proximal end 11 of the housing.

Preparation of the medicament delivery device is done by moving, e.g. by turning, the proximal housing section 13 and the distal housing section 14 in relation to each other. The turning movement will, due to the threads on the outside of the proximal housing section and corresponding threads on the inside surface of the distal housing section, move the distal housing section, and the plunger rod, towards the proximal housing section and transfer the diluent in the distal chamber 22 to the proximal chamber 21 of the container 20, thereby mixing the constituents of the two chambers. Preparation can also be achieved by the drive mechanism that generates a first force in the proximal direction of the medicament delivery device 10. The first force is transferred from the drive mechanism to the plungers 26 and 23 via the elongated plunger rod 30.

The drive mechanism (not illustrated), is of conventional type used in this type of medicament delivery devices. The drive mechanism is configured to generate the movement of the plunger rod 30 e.g. by co-operation with external treads 31 arranged around the outer periphery of the plunger rod in the distal end of the plunger rod 30. The distal end of the plunger rod has a thinner section 32 and a protrusion 33 configured to engage with corresponding components of the drive mechanism. In the proximal end of the plunger rod 30 a spinner 34, rotatable around the axis A, is arranged to abut the distal plunger 26. The spinner 34 is advantageous since the drive mechanism generates a turning movement of the plunger rod 30 and the rotatable spinner 34 reduces the friction that otherwise would occur been between the first plunger 26 and the proximal end of the plunger rod 30. The spinner 34 has a circular cross section, viewed along the axis A, with a slightly smaller radius than the distal plunger 26 in order to not be in contact with the inner periphery of the container. The outer periphery of the spinner 34 is furthermore provided with an annular groove 35 extending around the entire periphery of the spinner 34 in order to house leaking fluid from the container and to prevent the fluid from flowing along the plunger rod 30 in the distal direction towards the drive mechanism.

Between the spinner 34 and the threads 31 the plunger rod 30 is provided with a number of cut-out portions 36. The cut-out portions 36 are axially separated along the plunger rod 30 in order to efficiently prevent that leaking fluid flows along the plunger rod 30 without interruption. The cut out portions should be axially separated in order to prevent the fluid from flowing between the cut-out portions towards the drive mechanism in the distal end of the medicament delivery device.

Each cut-out portion 36 has a depth that is less than the radius of the plunger rod in the area of the cut-out portion in order to ensure that enough material remains in a plunger rod core to be able to withstand drive forces of the drive mechanism exerted on the plunger rod upon activation of the device. In order to ensure sufficient axial strength of the plunger rod the illustrated embodiment of the plunger rod has a central core 37 with a circular cross section in which no cut-out portions are arranged.

Each cut-out portion has a peripheral width that is less than half of the outer periphery of the plunger rod in order to make room for identical cut-out portions 36 on opposite sides of the plunger rod. The peripheral width of the cut-out portions could however be increased up to the entire periphery of the plunger rod. Preferably the total peripheral width of the cut-out portions exceed half the total peripheral lenght of the plunger rod in the area of the cut-out portions in order to provide efficient fluid traps and to ensure that the fluid will end up in the cut-out portions instead of flowing along the plunger rod between said cut-out portions.

In the appended figures, one embodiment of cut-out portions is illustrated. The design of the cut-out portions could however be modified in a number of different ways. For example the number and size of the cut-out portions could be changed or the cut-out portions could be replaced by recesses distributed over the outer periphery of the plunger rod. The recesses could for example have a spherical shape, round shape, holes extending through the plunger rod as long as enough mateiral remains on the side of the holes, annular grooves around the outer periphery of the plunger rod etc. Furthermore different types of recesses and/or cut-out portions could be used in combination.

A further advantageous embodiment is achieved if the cut-out portions or recesses are filled by a fluid absorbing material that is able to absorb fluid flowing along the plunger rod and to ensure that the fluid is retained by the recesses and/or cut-out portions.

Even though different embodiments of the medicament delivery device according to the invention have been described, the invention is not limited to the disclosed embodiments that could be modified further, for example: the number, size, and shape of the cut-out portions could be changed as well as the design of the injector device.

## Claims

1. Medicament delivery device (10) elongated along a longitudinal axis (A) and having a proximal (11) end and a distal end (12), said device being intended for use in combination with a disposable container (20) which comprises at least one chamber for a liquid medicament encapsulated in the container by at least one movable plunger (23, 26), said device (10) comprises:
- an elongated plunger rod (30) configured to be in contact with the at least one movable plunger (23, 26);
- a drive mechanism configured to, when the medicament delivery device is activated, apply a force in a proximal direction via said plunger rod (30) acting on said at least one movable plunger (23, 26);
- an elogated housing comprising a proximal housing section (13) and a distal housing section (14), said proximal housing section (13) being configured to accomodate the container (20) and said distal housing section (14) being configured to accomodate the plunger rod (30) and the driving mechanism; the device being **characterized in that** said elongated plunger rod (30) comprises an outer periphery in which at least two axially separated recesses and/or cut-out portions (35, 36) are arranged for retaining leaking liquid medicament that may flow along the plunger rod (30) to the drive mechanism, and wherein said axially separated recesses and/or cut-out portions (35, 36) of the plunger rod (30) are arranged at different locations around the outer periphery of the plunger rod (30).

2. Medicament delivery device (10) according to claim 1, wherein the disposable container (20) is a multi-chamber medicament container comprising a proximal chamber (21) wherein an active medicament is encapsulated, a distal chamber (22) wherein a diluent is encapsulated, a passage between the chambers (21, 22), a proximal movable plunger (23) sealing the proximal chamber and the passage, and a distal movable plunger (26) sealing the distal chamber and wherein the proximal end of elongated plunger rod (30) is configured to be in contact with the distal movable plunger (26).

3. Medicament delivery device (10) according to claim 2, wherein the device is configured such that when a force is exerted on said distal movable plunger (26) by the plunger rod (30) which is driven by the drive mechanism, said distal and proximal plungers (23, 26) are forced to move towards the proximal end (11) of the device such that the diluent may flow through the passage for being mixed with the active medicament forming a liquid medicament.

4. Medicament delivery device (10) according to anyone of the preceeding claims 1-3 wherein a spinner (34) is arranged rotatable around the longitudinal axis and in relation to the plunger rod (30), at the the proximal end of said plunger rod (30), said spinner (34) having an outer periphery provided with one or more recesses (35) and/or cut-out portions.

5. Medicament delivery device (10) according to any of claims 1-3, wherein at least two recesses and/or cut-out portions (36) in said plunger rod (30) extend a radial distance from the outer periphery of the plunger rod (30) towards the longitudinal axis (A) that is less than the radius of the plunger rod (30) in the area of the recess or cut-out portion (36).

6. Medicament delivery device (10) according to anyone of the previous claims, wherein said recesses and/or cut-out portions (35, 36) in the plunger rod (30) and/or spinner (34) are filled with an liquid absorbing material.

7. Medicament delivery device (10) according to anyone of the previous claims, wherein the peripheral width of said recesses and/or cut-out portions (36) in the plunger rod (30) exceeds half the peripheral width of the plunger rod (30) in the area of the recesses and/or cut-out portions (36).

8. Medicament delivery device (10) according to anyone of the previous claims, wherein device is an injector or an inhaler.

## Patentansprüche

1. Medikamentenabgabevorrichtung (10), die entlang einer Längsachse (A) verlängert ist und ein proximales (11) Ende und ein distales Ende (12) aufweist, wobei die Vorrichtung zur Verwendung in Kombination mit einem Einwegbehälter (20) vorgesehen ist, der mindestens eine Kammer für ein flüssiges Medikament umfasst, das in dem Behälter durch mindestens einen beweglichen Kolben (23, 26) eingekapselt ist, wobei die Vorrichtung (10) umfasst:
- eine verlängerte Kolbenstange (30), die dazu konfiguriert ist, mit dem mindestens einen beweglichen Kolben (23, 26) in Kontakt zu stehen;
- einen Antriebsmechanismus, der dazu konfiguriert ist, dass er, wenn die Medikamentenabgabevorrichtung aktiviert wird, eine Kraft in einer proximalen Richtung über die Kolbenstange (30) ausübt, die auf den mindestens einen beweglichen Kolben (23, 26) wirkt;
- ein verlängertes Gehäuse, das einen proximalen Gehäuseabschnitt (13) und einen distalen Gehäuseabschnitt (14) umfasst, wobei der proximale Gehäuseabschnitt (13) dazu konfiguriert ist, den Behälter (20) aufzunehmen, und der distale Gehäuseabschnitt (14) dazu konfiguriert ist, die Kolbenstange (30) und den Antriebsmechanismus aufzunehmen; wobei die Vorrichtung **dadurch gekennzeichnet ist, dass** die verlängerte Kolbenstange (30) einen Außenumfang umfasst, in dem mindestens zwei axial getrennte Aussparungen und/oder Ausschnitte (35, 36) zum Zurückhalten von auslaufendem flüssigen Medikament angeordnet sind, das entlang der Kolbenstange (30) zu dem Antriebsmechanismus fließen kann, und wobei die axial getrennten Aussparungen und/oder Ausschnitte (35, 36) der Kolbenstange (30) an unterschiedlichen Stellen um den Außenumfang der Kolbenstange (30) angeordnet sind.

2. Medikamentenabgabevorrichtung (10) nach Anspruch 1, wobei der Einwegbehälter (20) ein Mehrkammermedikamentenbehälter ist, umfassend eine proximale Kammer (21), in der ein aktives Medikament eingekapselt ist, eine distale Kammer (22), in der ein Verdünnungsmittel eingekapselt ist, einen Durchgang zwischen den Kammern (21, 22), einen proximalen beweglichen Kolben (23), der die proximale Kammer und den Durchgang abdichtet, und einen distalen beweglichen Kolben (26), der die distale Kammer abdichtet, und wobei das proximale Ende der verlängerten Kolbenstange (30) dazu konfiguriert ist, mit dem distalen beweglichen Kolben (26) in Kontakt zu stehen.

3. Medikamentenabgabevorrichtung (10) nach Anspruch 2, wobei die Vorrichtung so konfiguriert ist, dass, wenn eine Kraft auf den distalen beweglichen Kolben (26) durch die Kolbenstange (30), die durch den Antriebsmechanismus angetrieben wird, ausgeübt wird, der distale und der proximale Kolben (23, 26) dazu gezwungen werden, sich in Richtung des proximalen Endes (11) der Vorrichtung zu bewegen, sodass das Verdünnungsmittel durch den Durchgang zum Gemischtwerden mit dem aktiven Medikament fließen kann, wobei ein flüssiges Medikament gebildet wird.

4. Medikamentenabgabevorrichtung (10) nach einem der vorstehenden Ansprüche 1-3, wobei eine Schleudereinrichtung (34) drehbar um die Längsachse und in Bezug auf die Kolbenstange (30) an dem proximalen Ende der Kolbenstange (30) angeordnet ist, wobei die Schleudereinrichtung (34) einen Außenumfang aufweist, der mit einer oder mehreren Aussparungen (35) und/oder Ausschnitten versehen ist.

5. Medikamentenabgabevorrichtung (10) nach einem der Ansprüche 1-3, wobei sich mindestens zwei Aussparungen und/oder Ausschnitte (36) in der Kolbenstange (30) über einen radialen Abstand von dem Außenumfang der Kolbenstange (30) in Richtung der Längsachse (A) erstrecken, der kleiner als der Radius der Kolbenstange (30) in dem Bereich der Aussparung oder des Ausschnitts (36) ist.

6. Medikamentenabgabevorrichtung (10) nach einem der vorstehenden Ansprüche, wobei die Aussparungen und/oder Ausschnitte (35, 36) in der Kolbenstange (30) und/oder der Schleudereinrichtung (34) mit einem flüssigkeitsabsorbierenden Material gefüllt sind.

7. Medikamentenabgabevorrichtung (10) nach einem der vorstehenden Ansprüche, wobei die Umfangsbreite der Aussparungen und/oder Ausschnitte (36) in der Kolbenstange (30) die Hälfte der Umfangsbreite der Kolbenstange (30) in dem Bereich der Aussparungen und/oder Ausschnitte (36) übersteigt.

8. Medikamentenabgabevorrichtung (10) nach einem der vorstehenden Ansprüche, wobei eine Vorrichtung ein Injektor oder ein Inhalator ist.

## Revendications

1. Dispositif d'administration de médicament (10) allongé le long d'un axe longitudinal (A) et ayant une extrémité proximale (11) et une extrémité distale (12), ledit dispositif étant prévu pour une utilisation en combinaison avec un récipient jetable (20) qui comprend au moins une chambre pour un médicament liquide encapsulé dans le récipient par au moins un piston mobile (23, 26), ledit dispositif (10) comprend :
- une tige de piston allongée (30) configurée pour être en contact avec l'au moins un piston mobile (23, 26) ;
- un mécanisme d'entraînement configuré pour, lorsque le dispositif d'administration de médicament est activé, appliquer une force dans une direction proximale par l'intermédiaire de ladite tige de piston (30) agissant sur ledit au moins un piston mobile (23, 26) ;
- un logement allongé comprenant une section de logement proximale (13) et une section de logement distale (14), ladite section de logement proximale (13) étant configurée pour recevoir le récipient (20) et ladite section de logement distale (14) étant configurée pour recevoir la tige de piston (30) et le mécanisme d'entraînement ; le dispositif étant **caractérisé en ce que** ladite tige de piston allongée (30) comprend une périphérie externe dans laquelle au moins deux évidements et/ou parties de découpe (35, 36) axialement séparés sont agencés destinée à retenir un médicament liquide qui fuit qui peut s'écouler le long de la tige de piston (30) vers le mécanisme d'entraînement, et dans lequel lesdits évidements et/ou parties de découpe (35, 36) axialement séparés de la tige de piston (30) sont agencés au niveau d'emplacements différents autour de la périphérie externe de la tige de piston (30).

2. Dispositif d'administration de médicament (10) selon la revendication 1, dans lequel le récipient jetable (20) est un récipient de médicament à chambres multiples comprenant une chambre proximale (21) dans laquelle un médicament actif est encapsulé, une chambre distale (22) dans laquelle un diluant est encapsulé, un passage entre les chambres (21, 22), un piston mobile proximal (23) scellant la chambre proximale et le passage, et un piston mobile distal (26) scellant la chambre distale et dans lequel l'extrémité proximale de la tige de piston allongée (30) est configurée pour être en contact avec le piston mobile distal (26).

3. Dispositif d'administration de médicament (10) selon la revendication 2, dans lequel le dispositif est configuré de telle sorte que lorsqu'une force est exercée sur ledit piston mobile distal (26) par la tige de piston (30) qui est entraînée par le mécanisme d'entraînement, lesdits pistons distal et proximal (23, 26) sont forcés à se déplacer en direction de l'extrémité proximale (11) du dispositif de telle sorte que le diluant peut s'écouler à travers le passage pour être mélangé au médicament actif en formant un médicament liquide.

4. Dispositif d'administration de médicament (10) selon l'une quelconque des revendications 1 à 3 précédentes dans lequel une centrifugeuse (34) est agencée rotative autour de l'axe longitudinal et par rapport à la tige de piston (30), au niveau de l'extrémité proximale de ladite tige de piston (30), ladite centrifugeuse (34) ayant une périphérie externe pourvue d'un ou plusieurs évidements (35) et/ou parties de découpe.

5. Dispositif d'administration de médicament (10) selon l'une quelconque des revendications 1 à 3, dans lequel au moins deux évidements et/ou parties de découpe (36) dans ladite tige de piston (30) s'étendent sur une distance radiale à partir de la périphérie externe de la tige de piston (30) en direction de l'axe longitudinal (A) qui est inférieure au rayon de la tige de piston (30) dans la zone de l'évidement ou de la partie de découpe (36).

6. Dispositif d'administration de médicament (10) selon l'une quelconque des revendications précédentes, dans lequel lesdits évidements et/ou parties de découpe (35, 36) dans la tige de piston (30) et/ou la centrifugeuse (34) sont remplis avec un matériau absorbant liquide.

7. Dispositif d'administration de médicament (10) selon l'une quelconque des revendications précédentes, dans lequel la largeur périphérique desdits évidements et/ou parties de découpe (36) dans la tige de piston (30) dépasse la moitié de la largeur périphérique de la tige de piston (30) dans la zone des évidements et/ou parties de découpe (36).

8. Dispositif d'administration de médicament (10) selon l'une quelconque des revendications précédentes, dans lequel dispositif est un dispositif d'injection ou un inhalateur.
